# EUROPÄISCHE PATENTSCHRIFT

(11) **EP 2 147 645 B1**
(45) Veröffentlichungstag und Bekanntmachung des Hinweises auf die Patenterteilung: **31.12.2014**
(21) Anmeldenummer: 09166104.1
(22) Anmeldetag: 22.07.2009
(51) Int. Cl.: A61B 17/34

(54) **Medizinisches Instrument mit seitlich versetzbarer Dichtung**
Medical instrument with laterally mobile seal
Instrument médical doté d'un joint déplaçable latéralement

(30) Priorität: 23.07.2008 DE 102008035311
(43) Veröffentlichungstag der Anmeldung: 27.01.2010
(73) Patentinhaber: Karl Storz GmbH & Co. KG, 78532 Tuttlingen (DE)
(72) Erfinder: Sauer, Michael, 78532, Tuttlingen (DE); Teichtmann, Elmar, 75031 Eppingen (DE)
(74) Vertreter: Witte, Weller & Partner Patentanwälte mbB

(56) Entgegenhaltungen:
- EP-A1- 1 582 158
- WO-A1-99/52577
- US-A- 5 342 315

## Beschreibung

Die vorliegende Erfindung betrifft ein medizinisches Instrument, mit einer Kanüle, durch die Schäfte weiterer Instrumente mit unterschiedlichen Schaftdurchmessern hindurchführbar sind, mit einer Dichtung, die eine Öffnung mit veränderbarem Öffnungsquerschnitt aufweist, durch den die Schäfte mit unterschiedlichen Schaftdurchmessern dicht hindurchführbar sind, mit einer Spreizvorrichtung zum Spreizen der Öffnung der Dichtung, wobei die Spreizvorrichtung einen Spreizkonus aufweist, der mehrere Lamellen aufweist, die schwenkbar an einem Ringkörper angebracht sind und einen sich von proximal nach distal verjüngenden Spreizkörper bilden, der distal mit der Dichtung im Bereich der Öffnung in Verbindung steht, wobei die Dichtung und die Spreizvorrichtung in einem flexiblen Hüllkörper aufgenommen sind, so dass ein seitlicher Versatz eines eingeführten Schafts ausgeglichen wird und somit nicht zu einer Undichtigkeit führt.

Ein solches Instrument ist aus der EP 1 582 158 A1 bekannt.

Hierbei dienen mehrere sich überlappende flächige Schutzelemente dazu, eine distal nachfolgende Dichtung bei Einführen eines weiteren Instruments zu schützen. Dieses System aus Dichtungen und Schutzelementen ist in einem Balg aufgenommen und durch diesen mit dem Kanülenteil des medizinischen Instruments verbunden, so dass eine radiale Bewegung des Systems möglich wird.

Ein ähnliches medizinisches Instrument ist aus der EP 0 696 459 B1 bekannt.

Bei diesem medizinischen Instrument handelt es sich um einen Trokar, der in der minimal-invasiven Chirurgie eingesetzt wird. Dabei wird der entsprechende Trokar durch eine kleine Öffnung, die z.B. durch einen kleinen Schnitt herbeigeführt werden kann, mittels eines Trokardorns in den Körper des Patienten eingebracht. Nach Entfernen des Trokardorns werden durch diesen Trokar dann Schäfte von anderen Instrumenten, die für den chirurgischen Eingriff verwendet werden, eingeführt. Zu diesen Instrumenten zählen z.B. Greifwerkzeuge, Koagulationsinstrumente, Endoskope und dergleichen. Diese weisen unterschiedliche Durchmesser auf.

Da solche minimal-invasiven Eingriffe häufig unter Insufflation des Innenraums, in dem die Operation stattfindet, durchgeführt werden, müssen alle Eingriffsstellen gasdicht sein.

Bei der EP 0 696 459 B1 ist dazu eine elastische kegelförmige Dichtung in dem Trokar angeordnet, die sich von proximal nach distal verjüngt. Um eine gleichmäßige Aufweitung der Dichtung zu ermöglichen, wenn ein Instrument in den Trokar eingeführt wird, ist proximal von dieser Dichtung der Spreizkonus angeordnet. Ein in den Spreizkonus eingeführtes Instrument spreizt diesen radial und dieser weitet die Öffnung in der Dichtung auf. Neben der einfacheren Aufweitung der Dichtung verhindert der Spreizkonus ferner die Beschädigung der Dichtung beim Einführen von scharfkantigen Instrumenten, weil er aus einem harten Material hergestellt werden kann. Auf diese Weise wird die Dichtung vom Spreizkonus geschützt.

Beim Einsatz eines Instruments mit einem Schaftdurchmesser, der etwa dem der Kanüle des Trokars entspricht, wird die Öffnung der Dichtung mittig von diesem durchdrungen und maximal gespreizt, so dass der Schaft gleichmäßig um seinen ganzen Umfang mit der Dichtung in Kontakt kommt. Dadurch ist das Trokarsystem nach proximal abgedichtet.

Beim Einsatz eines Instruments mit einem schmaleren Schaft kann es zu einem Versatz von dem mittigen Durchgang durch die Öffnung der Dichtung kommen. Ferner kann es insbesondere bei schmaleren biegbaren Schäften zu einem schrägen Verlauf des Schaftes im Trokar kommen.

Beides führt dazu, dass die Dichtung nicht gleichmäßig um den ganzen Umfang des Schaftes mit diesem in Kontakt kommt. Die Folge sind unerwünschte Undichtigkeiten. Diese Führen zu einem Entweichen des für die Operation eingeströmten Gases und folglich zu einem Verlust der Insufflation.

Ein Durchführen von Instrumenten mit schmalen Schäften kann somit problematisch und erschwert verlaufen.

Es ist daher Aufgabe der vorliegenden Erfindung, ein medizinisches Instrument der Art, wie sie aus der EP 0 696 459 B1 bekannt ist, dahingehend weiter zu entwickeln, dass ein seitlicher Versatz eines eingeführten Schafts nicht zu einer Undichtigkeit am Trokar führen kann.

Erfindungsgemäß wird die Aufgabe dadurch gelöst, dass der flexible Hüllkörper ein flexibles Hüllrohr ist, das aus einer im Wesentlichen parallelen Aneinanderreihung von Ringelementen besteht.

Der Zusammenbau aus dem Spreizkonus aus steifem Material und der Dichtung aus elastischem Material stellt ein in seitlicher Richtung relativ instabiles Gebilde dar. Dies ist insbesondere bei der Ausgestaltung ausgeprägt, bei der sich Spreizkonus und Dichtung ausgehend von der Verbindungsstelle untereinander diametral voneinander weg erstrecken. Ein seitlich versetzt oder schräg in den Spreizkonus eingeführtes Instrument würde ein seitliches Ausweichen oder einen seitlichen Versatz des Zusammenbaus bewirken. Der Hüllkörper wirkt dem entgegen, lässt aber eine gewisse Flexibilität zu.

Bei einem ungleichmäßigen Kontakt zwischen einem eingeführten Schaft und der Öffnung der Dichtung verschiebt sich das Dichtungssystem so, dass der Schaft wieder mittig durch die Öffnung der Dichtung verläuft. Dadurch kommt die Dichtung wieder gleichmäßig auf dem ganzen Umfang des Schaftes mit diesem in Kontakt, wodurch die gewünschte Dichtigkeit des Trokarsystems erhalten wird. Das Hüllrohr bewirkt eine Art Aufrichtung oder Ausrichtung des Dichtungssystems in die geradlinig gestreckte Ausrichtung.

Somit gibt der Hüllkörper dem System aus Spreizkonus und Dichtung eine erhöhte Stabilität, welche insbesondere bei der Ausgestaltungsform, in der sich die Dichtung vom distalen Ende des Spreizkonus nach distal weg erstreckt, fehlt. Das liegt daran, dass der proximal gelegene Spreizkonus zunächst nur am proximalen Ende der Dichtung angeordnet ist. Da die Dichtung aber sehr beweglich ist, wird dadurch auch der Spreizkonus und folglich auch die Einlassöffnung in den Trokar sehr beweglich. Dies erschwert ein Einführen eines Schaftes in den Trokar und kann auch durch Schrägstellungen der Dichtung die Dichteigenschaften des Dichtungssystems negativ beeinflussen. Dem wirkt der Hüllkörper entgegen, indem er eine zusätzliche Verbindung des proximalen Endes des Spreizkonus mit dem Trokar herstellt und die Beweglichkeit des Dichtungssystems somit auf ein gewünschtes Maß reduziert.

Ein Vorteil bei der Verwendung von Ringelementen liegt darin, dass diese wenig anspruchsvoll zu fertigende Bauteile sind, was die Herstellung einfach macht.

In einer weiteren Ausgestaltung der Erfindung sind zwei benachbarte Ringelemente an zwei sich radial gegenüberliegenden Stellen miteinander verbunden und weiterhin die Verbindungsstellen eines Ringelements zu dem distal nächsten Ringelement jeweils um 90° gegenüber den Verbindungsstellen zu dem proximal nächsten Ringelement kreisförmig versetzt angeordnet.

Die abwechselnd um 90° kreisförmig versetzt angeordneten Verbindungsstellen zwischen den Ringelementen erlauben eine Flexibilität des Hüllrohrs in alle seitlichen Richtungen, da aufgrund der radial gegenüberliegenden Anordnung der Verbindungsstellen die zusammengehörigen Ringelemente eine Kippachse aufweisen. Durch diese können sie aus der zueinander parallelen Anordnung an umfänglich zwischen den Verbindungsstellen liegenden Stellen aufeinander zu gekippt werden. Der Versatz um 90° von einem zum nächsten Ringelementpaar sorgt in der Gesamtanordnung der Ringelemente dafür, dass ein Abkippen in zwei senkrecht zueinander verlaufende Richtungen möglich ist. Das heißt, der so erzeugte Hüllkörper kann an seinem freien proximalen Ende in zwei voneinander unabhängige Richtungen und somit in der durch diese Richtungen gegebenen Ebene bewegt werden. Neben der guten Beweglichkeit und der damit verbundenen Fähigkeit, einen Versatz eines Schaftes im Trokar auszugleichen, wird durch die um 90° zueinander versetzt angeordneten Verbindungsstellen gleichzeitig eine gute axiale Stabilität erreicht, da im Belastungsfall eine Gleichverteilung der einwirkenden Kräfte vorliegt.

In einer Ausgestaltung der Erfindung sind die Verbindungen flexible Stege.

Der Vorteil dieser Ausgestaltung besteht darin, dass die schmalen und damit flexiblen Stege direkt ohne mehrteilige Gelenke an die jeweiligen Elemente angeordnet sind. Dadurch kann das so aufgebaute flexible Hüllrohr als ein einziges Werkstück z.B. aus Kunststoff durch ein Spritzgussverfahren hergestellt werden. Da hierdurch ein nachträgliches Montieren der Ringelemente entfällt, erfolgt die Herstellung sehr schnell und einfach.

In einer Ausgestaltung der Erfindung sind die Verbindungen Kippgelenke.

Durch die Konstruktion des flexiblen Hüllrohrs aus einzelnen Ringelementen und durch deren Verbindung mittels Kippgelenken wird die Länge des flexiblen Hüllrohrs variabel. Sie kann somit bei der Konstruktion eines Dichtungssystems an die Länge des Spreizkonus und an die Länge der Dichtung angepasst werden. Mit einem Ring-elementtyp können somit unterschiedlich lange Hüllrohre aufgebaut werden, was diese Ausgestaltungsform sehr variabel macht.

In einer Ausgestaltung der Erfindung sind die Ringelemente an den Stellen einer Kippgelenkfassung höher als an denen eines Kippgelenkeinsatzes.

Die Stelle der Kippgelenkfassung im Ringelement muss, um den kompletten Kippgelenkeinsatz aufnehmen zu können, eine gewisse Höhe aufweisen. Diese Höhe ist an den übrigen Stellen des Ringelements geringer. Dadurch wird der Bereich, in dem die Ringelemente zueinander verkippt werden können, vergrößert, da der Abstand zwischen den Ringelementen an den umfänglich zwischen den Kippgelenken liegenden Stellen größer ist. Auf diese Weise wird ein größtmöglicher Neigungswinkel erreicht, was die Flexibilität des Hüllrohrs erhöht. Dadurch kann sich das Dichtungssystem besser an jeglichen Versatz eines Schaftes in der Öffnung der Dichtung anpassen.

In einer weiteren Ausgestaltung der Erfindung weitet sich der flexible Hüllkörper von proximal nach distal konisch auf.

Durch diese Aufweitung wird der Hüllkörper an den Durchschnittsunterschied zwischen dem Ringkörper des Spreizkonus und dem zumeist durchschnittsgrößeren distalen Ende der Dichtung angepasst. Dadurch wird einerseits ein guter Halt am distalen Ende der Dichtung und dem Trokargehäuse, an dem diese angeordnet ist, und andererseits am Spreizkonus sichergestellt. Dies führt zu der gewünschten Stabilität.

In einer weiteren Ausgestaltung erstreckt sich die Dichtung vom distalen Ende des Spreizkonus nach distal weg.

Die Wahl dieser konstruktiven Anordnung hat den Vorteil, dass zum einen das Zusammenfügen von Spreizkonus und Dichtung einfach vonstatten geht, da beide Bauteile über gut zugängliche Außenseiten miteinander verbunden werden. Zum anderen unterstützt die dabei vorliegende Anordnung der Dichtung die Entnahme von Gewebeproben, da sie auf diese Weise eine trichterartige Form darstellt, die von distal nach proximal verjüngt. Wird z.B. mit einem Greifwerkzeug eine solche Probe genommen und anschließend durch die Trokarhülse in proximale Richtung geführt, so gelangt diese schließlich an das Dichtungssystem. Dort wird sie aufgrund des allmählich geringer werdenden Durchmessers der Dichtung zur proximalen schmalen Öffnung der Dichtung geführt und ggf. auch in der Form angepasst. Ein ungewolltes Hängenbleiben aufgrund einer abrupten Durchmesseränderung im Verlauf des Herausziehens der Probe wird somit vermieden.

In einer weiteren Ausgestaltung der Erfindung ist das distale Ende der Lamellen des Spreizkonus formschlüssig mit einer Fassung am Rand der Öffnung in der Dichtung verbunden.

Durch diese Verbindung des distalen Endes der Lamellen mit der Öffnung der Dichtung wird ein Verrutschen der Lamellen beim Spreizen der Dichtung verhindert. Somit wird einem möglichen Verkanten der Lamellen entgegengewirkt und ein gleichmäßiges Spreizen sichergestellt. Ferner führt die Verbindung dazu, dass sich die Lamellen aufgrund der Rückstellkraft der gespreizten elastischen Dichtung nach dem Entfernen eines Schaftes aus dem Trokar wieder zusammen mit der Dichtungsöffnung zurück in die Ausgangsposition bewegen.

In einer Ausgestaltung der Erfindung ist das distale Ende der Lamellen des Spreizkonus mit der Dichtung verklebt.

Ein Verkleben zwischen Lamellen und Öffnung der Dichtung sorgt für einen unlösbaren festen Kontakt zwischen den Bauteilen. Hierdurch wird ein ungewolltes Trennen der Bauteile verhindert, so dass die Lamellen stets an der Öffnung der Dichtung angeordnet sind. Das Verkleben ist als Einzelmaßnahme, aber auch in Kombination mit der formschlüssigen Verbindung denkbar.

In einer weiteren Ausgestaltung der Erfindung besteht der Spreizkonus aus acht bis zwölf, vorzugsweise aus zehn Lamellen.

Die Verwendung von acht bis zwölf bzw. bevorzugt zehn Lamellen ermöglicht einerseits ein gleichmäßiges Aufweiten der Öffnung der Dichtung, da die Anzahl der verwendeten Lamellen so groß ist, dass die beim Aufweiten entstehenden Zwischenräume möglichst klein sind und außerdem der Abstand zwischen zwei Lamellen im gespreizten Zustand klein ist. Somit wird auch im gespreizten Zustand durch die Lamellen immer noch eine gleichmäßige runde Form an der Öffnung der Dichtung gebildet. Dadurch wird insbesondere die Dichtigkeit an der Öffnung der Dichtung während und nach dem Einführen eines Schaftes begünstigt. Andererseits weisen diese Lamellen aber auch eine genügend große Stabilität auf, da sie so noch eine ausreichend große Breite besitzen. Somit werden sie nicht beim Einführen speziell scharfkantiger Instrumente beschädigt oder zerstört, wodurch weiterhin der Schutz der Dichtung gegeben ist.

In weiteren Ausgestaltungen der Erfindung sind der Spreizkonus und der flexible Hüllkörper aus Hartplastik gefertigt.

Die Verwendung von Hartplastik für den Spreizkonus und den flexiblen Hüllkörper stellt im vorliegenden Fall einen guten Kompromiss dar, da einerseits eine ausreichende Stabilität, z.B. beim Einführen von scharfkantigen Instrumenten, erhalten wird und trotzdem eine einfache Herstellung, beispielsweise durch das Spritzgusverfahren, ermöglicht wird.

In einer weiteren Ausgestaltung der Erfindung ist die Dichtung aus einem elastischen Material gefertigt.

Die Verwendung eines elastischen Materials für die Dichtung ermöglicht einerseits ein geeignetes Aufweiten der Öffnung der Dichtung durch den Spreizkonus beim Einführen des Schaftes eines Instruments und andererseits eine gleichzeitig optimale Abdichtung am Schaft eines solchen Instruments aufgrund des dichten Kontakts zwischen elastischem Material und Schaft. Dieser wird insbesondere dadurch erreicht, dass die gespreizte Öffnung der Dichtung aufgrund der Rückstellkraft des elastischen Materials gegen den eingeführten Schaft umlaufend gepresst wird.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand einiger ausgewählter Ausführungsbeispiele in Zusammenhang mit den beiliegenden Zeichnungen näher beschrieben und erläutert. Es zeigen:
- Fig. 1: eine perspektivische Seitenansicht eines medizinischen Instruments mit variabler Dichtung,
- Fig. 2: einen Schnitt längs der Linie II-II aus Fig. 1,
- Fig. 3: ein Dichtungssystem des medizinischen Instruments von proximal betrachtet,
- Fig. 4: eine perspektivische Darstellung eines flexiblen Hüllrohrs des Dichtungssystems,
- Fig. 5: einen Schnitt längs der Linie V-V aus Fig. 3,
- Fig. 6: eine perspektivische Darstellung eines Spreizkonus,
- Fig. 7: eine perspektivische Darstellung einer Dichtung,
- Fig. 8: eine perspektivische Darstellung eines flexiblen Hüllrohrs mit Kippgelenken,
- Fig. 9: eine perspektivische Ansicht eines Spreizkonus mit Kugelkopfaufweisenden Lamellen,
- Fig. 10: die Kugelkopf-aufweisende Lamelle in perspektivischer Einzelansicht,

Ein in den Figuren dargestelltes medizinisches Instrument ist in seiner Gesamtheit mit der Bezugsziffer 10 bezeichnet.

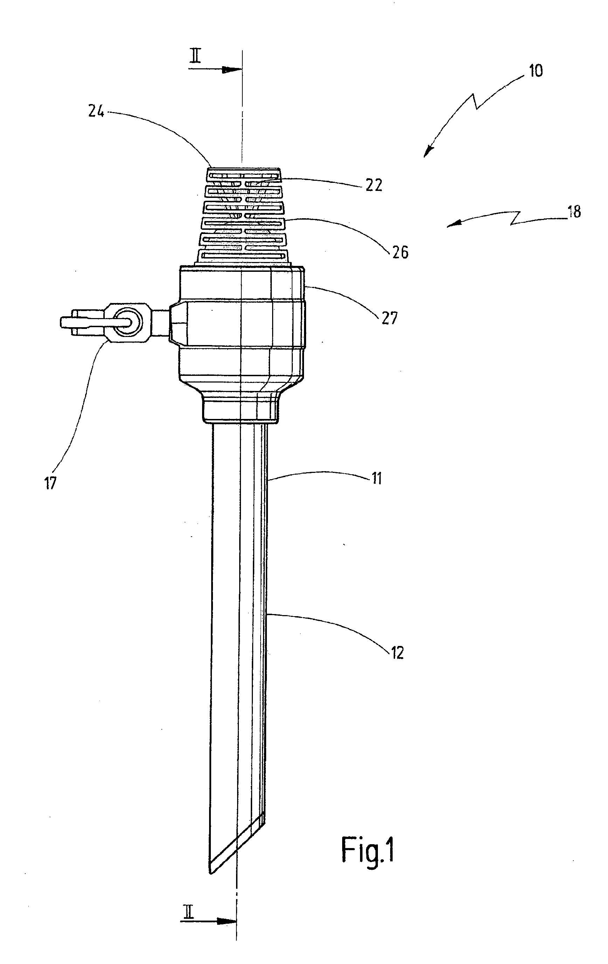

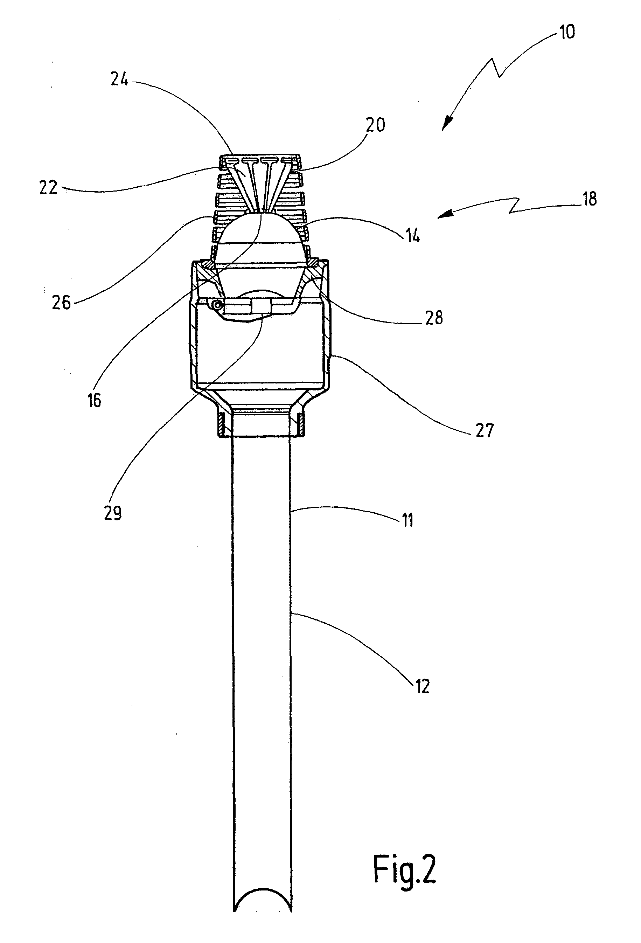

Das dargestellte medizinische Instrument 10 ist ein Trokar und weist distal eine Trokarhülse 11, die hier durch eine Kanüle 12 gebildet wird, mittig ein Trokargehäuse 27 und proximal an diesem angeordnet ein Dichtungssystem 18 auf. Fig. 1 zeigt ferner ein am Trokargehäuse 28 angeordnetes Ventil 17, welches z.B. als Zuleitung für Gase dienen kann. Diese werden dazu verwendet, eine Insufflation der Operationsstelle durchzuführen, um dadurch einen besseren Zugang zu den Organen, Gefäßen, Geweben oder dergleichen, an denen die Operation durchgeführt werden soll, zu erhalten. Um ein ungewolltes Austreten der Gase durch den Trokar in proximale Richtung zu verhindern, ist distal des Dichtungssystems 18 ein selbstschließender Verschluss 29 angeordnet (Fig. 2). Dieser verschließt die proximale Öffnung, sobald kein Schaft in das medizinische Instrument 10 eingeführt ist.

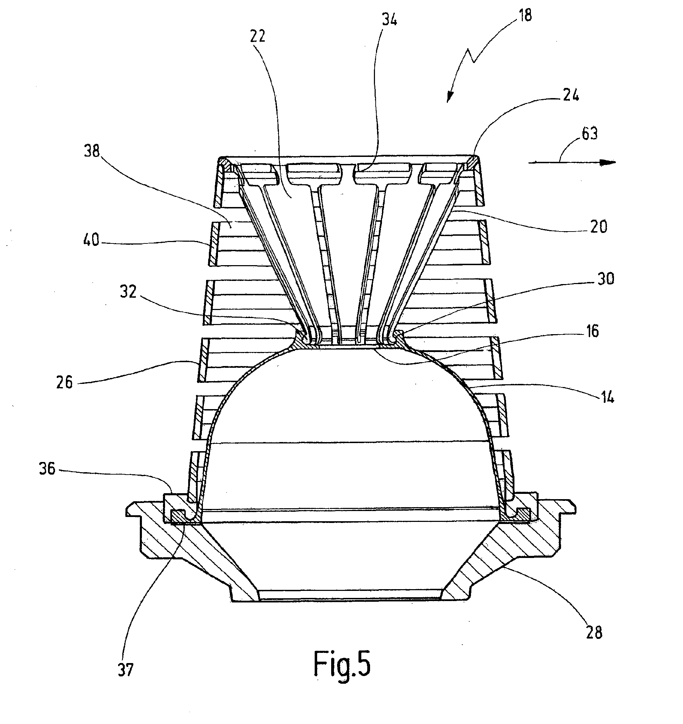

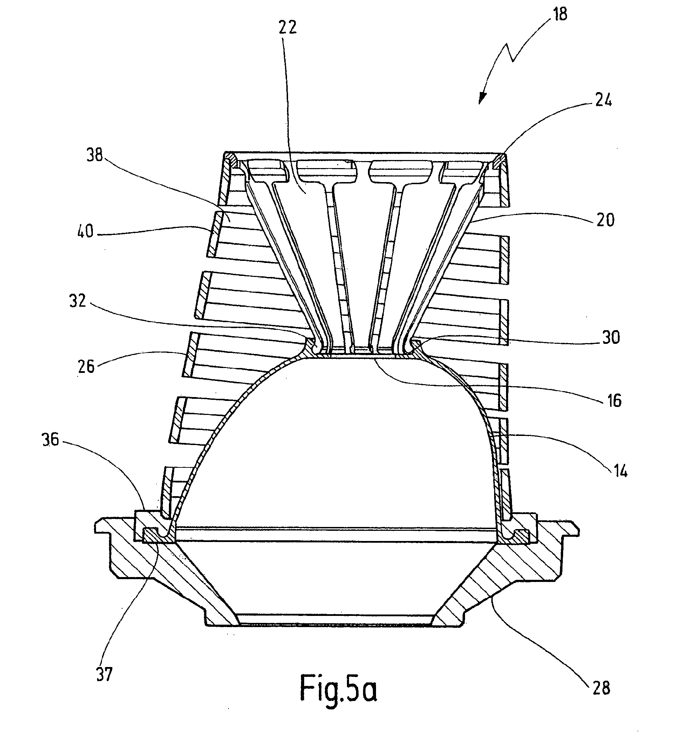

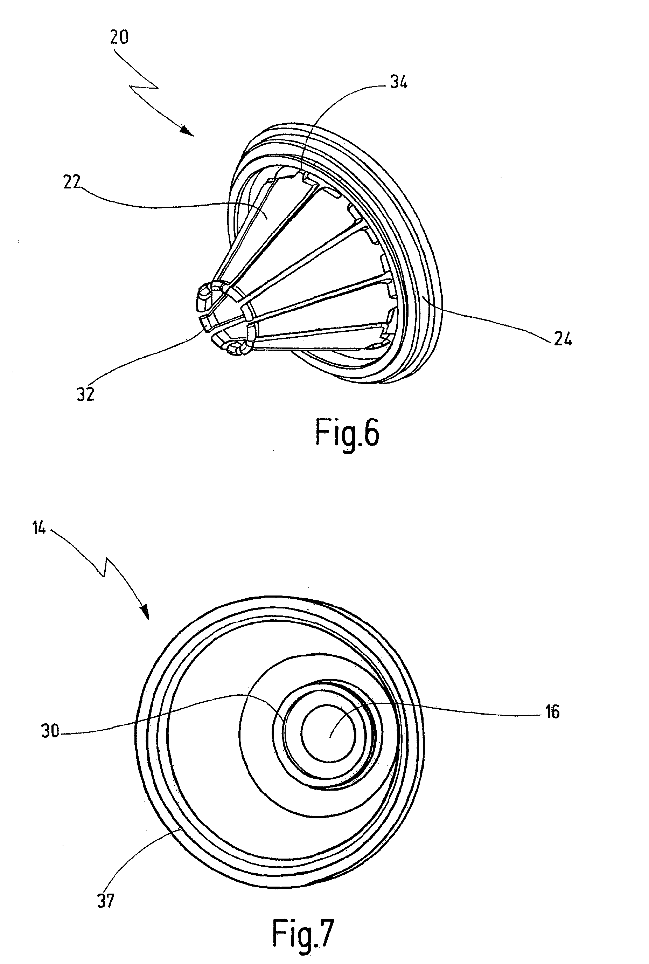

Wie in den Fig. 1, 2 und 5 zu sehen ist, weist das Dichtungssystem 18 eine Dichtung 14 auf. Diese ist mit einem Dichtungsrand 37 durch einen Befestigungsring 36 an einer Dichtungshalterung 28 befestigt, mit der das Dichtungssystem 18 am Trokargehäuse 27 angebracht ist. Sie erstreckt sich proximal von der Dichtungshalterung 28 weg und verjüngt dabei. An ihrem proximalen Ende weist die Dichtung 14 eine Öffnung 16 sowie eine diese Öffnung 16 umgebende Fassung 30 auf (Fig. 5 und 7). An der Fassung 30 befestigt und sich von der Dichtung 14 proximal weg erstreckend ist ein Spreizkonus 20 angeordnet (Fig. 5).

Wie in Fig. 5 und 6 zu sehen ist, ist dieser Spreizkonus 20 aus einem Ringkörper 24, an dem Lamellen 22 mittels Folienscharnieren 34 verschwenkbar ringförmig distal ausgerichtet angeordnet sind, aufgebaut. Diese Lamellen 22 weisen am distalen Ende Abschlüsse 32 auf, die formschlüssig mit der Fassung 30 der Dichtung 14 verbunden sind. Durch den Querschnittsunterschied zwischen dem Ringkörper 24 und der Fassung 30 erhält der Spreizkonus 20 seine konische Form. Die Lamellen 22 verjüngen von proximal nach distal in der Art, dass sich eine gleichmäßige nahezu geschlossene innere Fläche des Spreizkonus 20 im ungespreizten Zustand ergibt.

Durch die Verbindung zwischen den Lamellen 22 und der Dichtung 14, wobei die Abschlüsse 32 in der Fassung 30 eingelegt sind, werden bei Einführen eines Schaftes durch diesen zunächst die Lamellen 22 radial nach außen gedrückt, wodurch gleichzeitig die Öffnung 16 der Dichtung 14 gespreizt wird, ohne jedoch selber direkt durch den Schaft gespreizt zu werden.

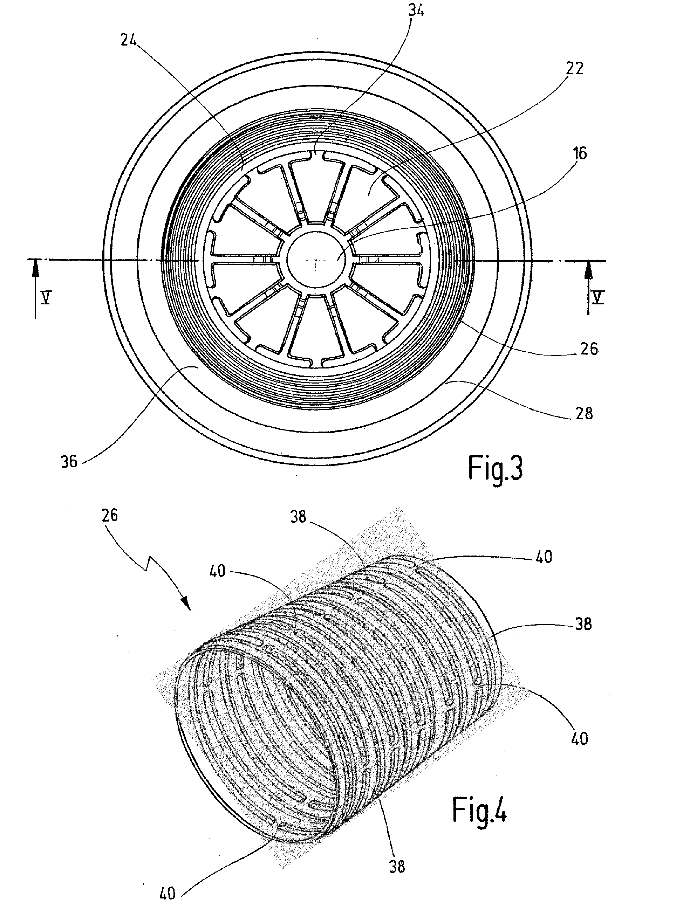

Das Gebilde aus der Dichtung 14 und dem Spreizkonus 20 wird von einem Hüllrohr 26 umhüllt. Dazu ist dieses Hüllrohr 26 distal mit der Dichtungshalterung 28 und proximal mit dem Ringkörper 24 verbunden (Fig. 5). Das Hüllrohr 26 besteht aus einzelnen Ringelementen 38, die über jeweils radial gegenüberliegende Stege 40 miteinander verbunden sind (Fig. 4). Um eine Flexibilität des Hüllrohrs zu erhalten, wird dieses einerseits aus einem ausreichend elastischen Material gefertigt, so dass eine Flexibilität der Stege 40 gegeben ist und die Stege 40 außerdem von einem zum nächsten Ringelementpaar jeweils um 90° kreisförmig versetzt zueinander angeordnet.

Wird z.B. durch den seitlichen Versatz eines durchschnittsgeringeren Schaftes eine Kraft auf das Dichtungssystem entsprechend der Richtung des Pfeils 63 ausgeübt, so bewegt sich der Spreizkonus 20 in solch einem beweglichen flexiblen Hüllrohr 26 mit dem Schaft mit (Fig. 5a). Durch die Bewegung des Spreizkonus 20 in Richtung des Pfeils 63 wird das proximale Ende des Hüllrohrs 26 in gleicher Weise bewegt. Der dadurch entstehende Versatz zwischen proximalem und distalem Ende des Hüllrohrs 26 wird durch dessen Flexibilität ausgeglichen, so dass es einen gebogenen Verlauf aufweist.

Die Verbindung zwischen den Lamellen 22 und der Fassung 30 sorgt bei einer solchen Bewegung des Spreizkonus 20 für eine entsprechende Verformung der Dichtung 14. Die Folge ist, dass der Schaft trotz des Versatzes eine zentrale Lage in der Öffnung 16 der Dichtung 14 aufweist und kein Verkanten in der Öffnung 16 sowie keine sich daraus ergebende Undichtigkeit auftritt. Durch das flexible Hüllrohr 26 passt sich das Dichtungssystem somit an jeglichen Versatz eines Schaftes im medizinischen Instrument an.

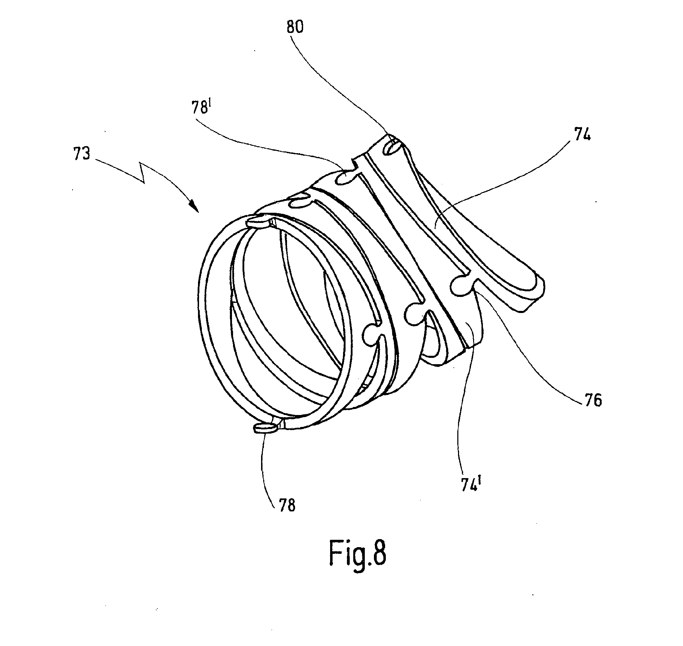

Ein weiteres Ausführungsbeispiel für ein flexibles Hüllrohr ist in Fig. 8 gezeigt. Im Gegensatz zu dem in Fig. 4 beschriebenen Hüllrohr 26, welches z.B. durch ein Spritzgussverfahren als ein einziges Teil hergestellt werden kann, zeigt Fig. 8 ein Hüllrohr 73, das aus separaten Ringelementen 74 aufgebaut ist. Diese Ringelemente 74 weisen zwei unterschiedliche Seiten auf. Während die eine Seite zwei Kippgelenkfassungen 80 aufweist, besitzt die andere Seite zwei Kippgelenkeinsätze 78. Die Kippgelenkeinsätze 78 und die Kippgelenkfassungen 80 sind für sich jeweils einander gegenüber angeordnet. Zueinander sind die Kippgelenkfassung 80 und der Kippgelenkeinsatz 78 an einem Ringelement 74 um 90° kreisförmig versetzt angeordnet.

Wie in Fig. 8 gezeigt, werden mehrere Ringelemente 74 durch Verbindung von den Kippgelenkeinsätzen 78 eines Rings mit den Kippgelenkfassungen 80 eines anderen Rings miteinander verbunden. Dadurch ergibt sich das Hüllrohr 73, welches mit seinen Verbindungen zwischen den Kippgelenkeinsätzen 78 und den Kippgelenkfassungen 80 den Stegen 40 zwischen den Ringelementen 38 des Hüllrohrs 26 ähnelt (Fig. 4 und Fig. 8). Der Kippgelenkeinsatz 78 und die Kippgelenkfassung 80 bilden so ein Kippgelenk 76, welches für die Flexibilität des Hüllrohrs 73 verantwortlich ist.

Um einen möglichst großen Beweglichkeitsbereich zu erhalten, ist dabei an den Kippgelenkfassungen 80 das Ringelement 74 höher als an den Stellen des Kippgelenkeinsatzes 78 (Fig. 8). Das Ringelement 74 kann somit weiter um die Achse, die sich durch die einander radial gegenüberliegenden Kippgelenke 76 bildet, verschwenkt werden. Ein erstes Ringelement 74 kann somit an der Stelle der Kippgelenkfassung 80 weiter zu einem zweiten Ringelement 74' verschwenkt werden, als wenn letzteres an der Stelle des Kippgelenkeinsatzes 78' die gleiche Höhe aufweisen würde wie an der Stelle des Kippgelenks 76.

Dadurch wird auch mit diesem Ausführungsbeispiel der gleiche Effekt bei seitlichem Versatz eines eingeführten Schaftes erzielt, wie er für das flexible Hüllrohr 26 in Zusammenhang mit Fig. 5 und Fig. 5a erläutert wurde.

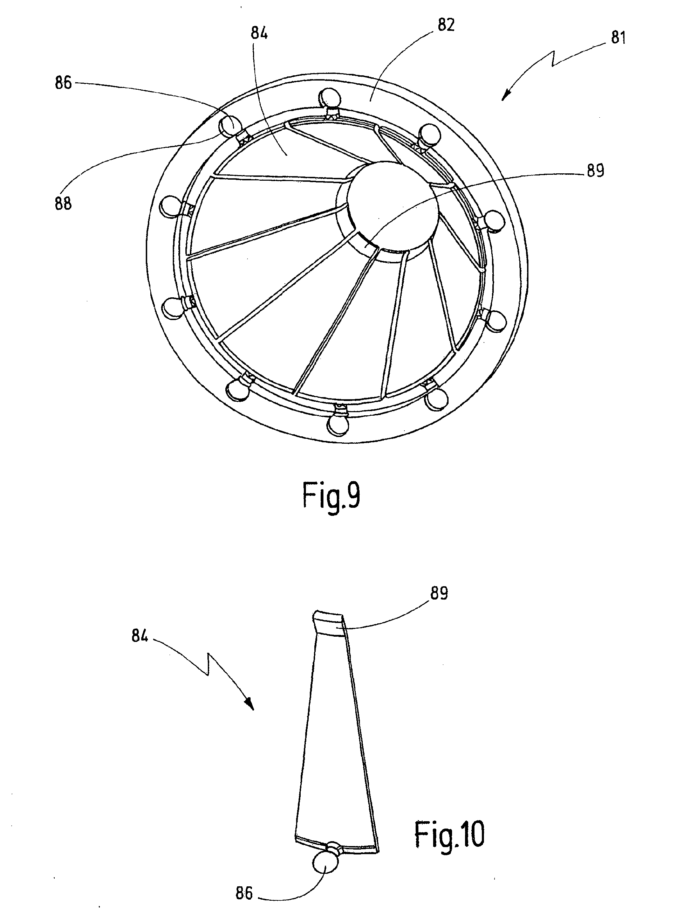

Ein anderes Ausführungsbeispiel für Lamellen ist in Fig. 10 dargestellt. Eine dort dargestellte Lamelle 84 weist am distalen Ende einen Abschluss 89 und am proximalen Ende einen Kugelkopf 86 auf.

Dieser Kugelkopf 86 dient zur beweglichen Verbindung der Lamelle 84 mit einem Ringkörper 82. Hierzu weist der Ringkörper 82 Öffnungen 88 auf, in die die Kugelköpfe 86 eingesetzt werden können. Das Einsetzen der Lamellen ist sowohl als Einrastmechanismus als auch als einfacher Einlegmechanismus denkbar. Für zumindest letztere Variante wäre dann noch ein hier nicht gezeigter Abdeckring nötig, der ein ungewolltes Herabfallen der Lamellen 84 aus dem Ringkörper 82 verhindert (Fig. 9).

Dadurch ergibt sich ein Spreizkonus 81, analog zu dem Spreizkonus 20 mit den Folienscharnieren 34 des zuvor erwähnten Ausführungsbeispiels. Analog zu diesem weisen auch die Lamellen 84 eine Verjüngung von proximal nach distal auf.

## Patentansprüche

1. Medizinisches Instrument, mit einer Kanüle (12) durch die Schäfte weiterer Instrumente mit unterschiedlichen Schaftdurchmessern hindurchführbar sind, mit einer Dichtung (14), die eine Öffnung (16) mit veränderbarem Öffnungsquerschnitt aufweist, durch den die Schäfte mit unterschiedlichen Schaftdurchmesser dicht hindurchführbar sind, mit einer Spreizvorrichtung zum Spreizen der Öffnung (16) der Dichtung (14), wobei die Spreizvorrichtung einen Spreizkonus (20, 81) aufweist, der mehrere Lamellen (22, 84) aufweist, die schwenkbar an einem Ringkörper (24, 82) angebracht sind und einen sich von proximal nach distal verjüngenden Spreizkörper bilden, der distal mit der Dichtung (14) im Bereich der Öffnung (16) in Verbindung steht, wobei die Dichtung (14) und die Spreizvorrichtung in einem flexiblen Hüllkörper aufgenommen sind, so dass ein seitlicher Versatz eines eingeführten Schafts ausgeglichen wird und somit nicht zu einer Undichtigkeit führt, **dadurch gekennzeichnet, dass** der flexible Hüllkörper ein flexibles Hüllrohr (26, 73) ist, das aus einer im Wesentlichen parallelen Aneinanderreihung von Ringelementen (38, 74) besteht.

2. Medizinisches Instrument nach Anspruch 1, **dadurch gekennzeichnet, dass** zwei benachbarte Ringelemente (38, 74) an zwei sich radial gegenüberliegenden Stellen verbunden sind.

3. Medizinisches Instrument nach Anspruch 2, **dadurch gekennzeichnet, dass** die Verbindungsstellen eines Ringelements (38, 74) zu dem distal nächsten Ringelement (38, 74) jeweils immer um 90° gegenüber den Verbindungsstellen zu dem proximal nächsten Ringelement (38, 74) kreisförmig versetzt angeordnet sind.

4. Medizinisches Instrument nach Anspruch 2 oder 3, **dadurch gekennzeichnet, dass** die Verbindungen flexible Stege (40) sind.

5. Medizinisches Instrument nach Anspruch 2 oder 3, **dadurch gekennzeichnet, dass** die Verbindungen Kippgelenke (76) sind.

6. Medizinisches Instrument nach Anspruch 5, **dadurch gekennzeichnet, dass** die Ringelemente (74) an den Stellen einer Kippgelenkfassung (80) höher sind als an denen eines Kippgelenkeinsatzes (78).

7. Medizinisches Instrument nach einem der Ansprüche 1 bis 6, **dadurch gekennzeichnet, dass** sich der flexible Hüllkörper von proximal nach distal konisch aufweitet.

8. Medizinisches Instrument nach einem der Ansprüche 1 bis 7, **dadurch gekennzeichnet, dass** sich die Dichtung (14) vom distalen Ende des Spreizkonus (20, 81) nach distal weg erstreckt.

9. Medizinisches Instrument nach einem der Ansprüche 1 bis 8, **dadurch gekennzeichnet, dass** das distale Ende der Lamellen (22, 84) des Spreizkonus (20, 81) formschlüssig mit einer Fassung am Rand der Öffnung (16) in der Dichtung (14) verbunden ist.

10. Medizinisches Instrument nach einem der Ansprüche 1 bis 9, **dadurch gekennzeichnet, dass** das distale Ende der Lamellen (22, 84) des Spreizkonus (20, 81) mit der Dichtung (14) verklebt ist.

11. Medizinisches Instrument nach einem der Ansprüche 1 bis 10, **dadurch gekennzeichnet, dass** der Spreizkonus (20, 81) aus acht bis zwölf Lamellen (22, 84) besteht.

12. Medizinisches Instrument nach einem der Ansprüche 1 bis 10, **dadurch gekennzeichnet, dass** der Spreizkonus (20, 81) aus zehn Lamellen (22, 84) besteht.

13. Medizinisches Instrument nach einem der Ansprüche 1 bis 12, **dadurch gekennzeichnet, dass** der Spreizkonus (20, 81) aus Hartplastik gefertigt ist.

14. Medizinisches Instrument nach einem der Ansprüche 1 bis 13, **dadurch gekennzeichnet, dass** der flexible Hüllkörper aus Hartplastik gefertigt ist.

15. Medizinisches Instrument nach einem der Ansprüche 1 bis 14, **dadurch gekennzeichnet, dass** die Dichtung (14) aus einem elastischen Material gefertigt ist.

## Claims

1. Medical instrument, with a cannula (12) through which shafts of other instruments having different shaft diameters can be guided, with a seal (14) comprising an opening (16) with a variable opening cross section through which the shafts having different shaft diameters can be guided sealingly, with an expansion device for expanding the opening (16) of the seal (14), which expansion device has an expansion cone (20, 81) with a plurality of slats (22, 84) that are mounted pivotably on an annular body (24, 82) and form an expansion body that narrows from the proximal end to the distal end and that is connected at the distal end to the seal (14) in the area of the opening (16), wherein the seal (14) and the expansion device are accommodated in a flexible casing structure, thereby leveling out a lateral offset of an inserted shaft and thereby avoiding a leak, **characterized in that** the flexible casing structure is a flexible casing tube (26, 73), which is composed of a substantially parallel series of ring elements (38, 74).

2. Medical instrument of Claim 1, **characterized in that** two adjacent ring elements (38, 74) are connected at two radially opposite sites.

3. Medical instrument of Claim 2, **characterized in that** the connecting sites connecting a ring element (38, 74) to the distally adjacent ring element (38, 74) are in each case always offset by 90° with respect to the connecting sites to the proximally adjacent ring element (38, 74).

4. Medical instrument of Claims 2 or 3, **characterized in that** the connections are flexible webs (40).

5. Medical instrument of Claims 2 or 3, **characterized in that** the connections are tilting joints (76).

6. Medical instrument of Claim 5, **characterized in that** the ring elements (74) are higher at the sites of a tilting-joint socket (80) than at those of a tilting-joint insert (78),

7. Medical instrument of anyone of Claims 1 through 6, **characterized in that** the flexible casing structure widens conically from the proximal end to the distal end.

8. Medical instrument of anyone of Claims 1 through 7, **characterized in that** the seal (14) extends in the distal direction away from the distal end of the expansion cone (20, 81).

9. Medical instrument of anyone of Claims 1 through 8, **characterized in that** the distal end of the slats (22, 84) of the expansion cone (20, 81) is connected with a form fit to a socket at the edge of the opening (16) in the seal (14).

10. Medical instrument of anyone of Claims 1 through 9, **characterized in that** the distal end of the slats (22, 84) of the expansion cone (20, 81) is adhesively bonded to the seal (14).

11. Medical instrument of anyone of Claims 1 through 10, **characterized in that** the expansion cone (20, 81) is composed of eight to twelve slats (22, 84).

12. Medical instrument of anyone of Claims 1 through 10, **characterized in that** the expansion cone (20, 81) is composed of ten slats (22, 84).

13. Medical instrument of anyone of Claims 1 through 12, **characterized in that** the expansion cone (20, 81) is made of hard plastic.

14. Medical instrument of anyone of Claims 1 through 13, **characterized in that** the flexible casing structure is made of hard plastic.

15. Medical instrument of anyone of Claims 1 through 14, **characterized in that** the seal (14) is made of an elastic material.

## Revendications

1. Instrument médical, comprenant une canule (12) à travers laquelle les tiges d'autres instruments présentant des diamètres de tige différents peuvent être insérées, comprenant une garniture d'étanchéité (14), laquelle présente une ouverture (16) ayant une section transversale modifiable, à travers laquelle les tiges présentant des diamètres de tige différents peuvent être insérées de manière étanche, comprenant un dispositif d'écartement destiné à écarter l'ouverture (16) de la garniture d'étanchéité (14), dans lequel le dispositif d'écartement présente un cône d'écartement (20, 81), lequel présente plusieurs lamelles (22, 84), lesquelles sont disposées de manière à être pivotables sur un corps de bague (24, 82) et forment un corps d'écartement se rétrécissant de l'extrémité proximale vers l'extrémité distale, lequel est relié de manière distale avec la garniture d'étanchéité (14) dans le secteur de l'ouverture (16), où la garniture d'étanchéité (14) et le dispositif d'écartement sont reçus dans un corps enveloppant flexible, de sorte qu'un décalage latéral d'une tige introduite est compensé et par conséquent n'engendre pas de fuites, **caractérisé en ce que** le corps enveloppant flexible est un tube de gainage flexible (26, 73), lequel se compose d'une série sensiblement parallèle d'éléments de bague (38, 74).

2. Instrument médical selon la revendication 1, **caractérisé en ce que** deux éléments de bague adjacents (38, 74) sont reliés au niveau de deux positions diamétralement opposées.

3. Instrument médical selon la revendication 2, **caractérisé en ce que** les positions de liaison d'un élément de bague (38, 74) destinées à l'élément de bague (38, 74) distal qui suit sont respectivement agencées en forme de cercle de manière à toujours être décalées à 90° par rapport aux positions de liaison destinées à l'élément de bague (38, 74) proximal qui suit.

4. Instrument médical selon la revendication 2 ou 3, **caractérisé en ce que** les liaisons sont des entretoises flexibles (40).

5. Instrument médical selon la revendication 2 ou 3, **caractérisé en ce que** les liaisons sont des articulations à basculement (76).

6. Instrument médical selon la revendication 5, **caractérisé en ce que** les éléments de bague (74) au niveau des positions d'un embout femelle d'articulation à basculement (80) sont plus hauts qu'au niveau des positions d'un embout mâle d'articulation à basculement (78).

7. Instrument médical selon l'une des revendications 1 à 6, **caractérisé en ce que** le corps enveloppant flexible s'évase de manière conique de l'extrémité proximale vers l'extrémité distale.

8. Instrument médical selon l'une des revendications 1 à 7, **caractérisé en ce que** la garniture d'étanchéité (14) s'étend depuis l'extrémité distale du cône d'écartement (20, 81) vers l'extrémité distale.

9. Instrument médical selon l'une des revendications 1 à 8, **caractérisé en ce que** l'extrémité distale de la lamelle (22, 84) du cône d'écartement (20, 81) est liée par coopération de formes avec un embout femelle au bord de l'ouverture (16) dans la garniture d'étanchéité (14).

10. Instrument médical selon l'une des revendications 1 à 9, **caractérisé en ce que** l'extrémité distale de la lamelle (22, 84) du cône d'écartement (20, 81) est collée à la garniture d'étanchéité (14).

11. Instrument médical selon l'une des revendications 1 à 10, **caractérisé en ce que** le cône d'écartement (20, 81) se compose de huit à douze lamelles (22, 84).

12. Instrument médical selon l'une des revendications 1 à 10, **caractérisé en ce que** le cône d'écartement (20, 81) se compose de dix lamelles (22, 84).

13. Instrument médical selon l'une des revendications 1 à 12, **caractérisé en ce que** le cône d'écartement (20, 81) est réalisé en matière plastique rigide.

14. Instrument médical selon l'une des revendications 1 à 13, **caractérisé en ce que** le corps enveloppant flexible est réalisé en matière plastique rigide.

15. Instrument médical selon l'une des revendications 1 à 14, **caractérisé en ce que** la garniture d'étanchéité (14) est réalisée en un matériau élastique.
